(19) Europäisches Patentamt
European Patent Office
Office européen des brevets

(11) Numéro de publication : **0 099 789**
**B1**

(12) **FASCICULE DE BREVET EUROPÉEN**

(45) Date de publication du fascicule du brevet : **29.07.87**

(51) Int. Cl.⁴ : **C 07 D453/02**, A 61 K 31/435

(21) Numéro de dépôt : **83401338.5**

(22) Date de dépôt : **29.06.83**

(54) **Nouveaux dérivés de l'amino-3 quinuclidine, leur procédé de préparation et leur application en thérapeutique.**

(30) Priorité : **02.07.82 FR 8211670**

(43) Date de publication de la demande :
**01.02.84 Bulletin 84/05**

(45) Mention de la délivrance du brevet :
**29.07.87 Bulletin 87/31**

(84) Etats contractants désignés :
**BE CH DE FR GB IT LI LU NL SE**

(56) Documents cités :
**FR-A- 2 329 266**
**US-A- 3 702 324**
**CHEMICAL ABSTRACTS, vol. 79, 1973, page 311, no. 146358a, Columbus, Ohio, USA, E.E. MIKHLINA et al.: "Synthesis and pharmacological study of 3-hydroxy- and 3-aminoquinuclidine derivatives"**
**CHEMICAL ABSTRACTS**
**Le dossier contient des informations techniques présentées postérieurement au dépôt de la demande et ne figurant pas dans le présent fascicule.**

(73) Titulaire : **DELALANDE S.A.**
**32, rue Henri Regnault**
**F-92400 Courbevoie (FR)**

(72) Inventeur : **Imbert, Thierry**
**8, Hameau de la Levrière**
**F-78590 Noisy-le-Roi (FR)**
Inventeur : **Dorme, Nicole**
**106, rue de Longchamp**
**F-75016 Paris (FR)**
Inventeur : **Langlois, Michel**
**4, place César Franck**
**F-78530 Buc (FR)**

(74) Mandataire : **Kedinger, Jean-Paul et al**
**c/o Cabinet Malemont 42, avenue du Président Wilson**
**F-75116 Paris (FR)**

**EP 0 099 789 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention a pour objet de nouveaux dérivés de l'amino-3 quinuclidine, leur procédé de préparation et leur emploi en thérapeutique.

D'après US-A-3 702 324, FR-A-2 329 266 et Chemical Abstracts, volume 79, 1973, page 311, n° 146 358 a, on connaît déjà des dérivés de benzamido-3 quinuclidine, présentant une activité thérapeutique.

Les nouveaux dérivés selon l'invention répondent, plus précisément à la formule générale :

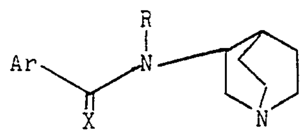

(I)

dans laquelle X représente un atome de soufre ou d'oxygène, R désigne un atome d'hydrogène, un groupe alkyle de 1 à 4 atomes de carbone ou le groupe benzyle, et Ar désigne :

un noyau phényle substitué par un atome d'halogène ou un groupe méthoxy,

un noyau phényle de structure

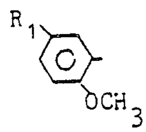

dans laquelle $R_1$ représente un atome d'halogène ou un groupe alkylcarbonyle dont le reste alkyle comporte de 1 à 4 atomes de carbone,

le groupe (fluoro-3 méthoxy-2) phényle,

le groupe (amino-2 méthoxy-4) pyrimidinyle-5, ou

un noyau phényle de structure

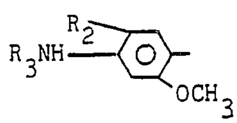

où $R_2$ représente un atome d'halogène et $R_3$ représente un atome d'hydrogène ou un groupe alkylcarbonyle dont le reste alkyle comporte de 1 à 4 atomes de carbone.

Du fait de la présence d'un carbone asymétrique dans leur molécule, les dérivés de formule (I) existent sous la forme de racémiques, d'isomères optiques dextrogyres et d'isomères optiques lévogyres ; la présente invention englobe par conséquent les composés de formule (I) sous forme racémique, sous forme d'isomères optiques dextrogyres et sous forme d'isomères optiques lévogyres.

La présente invention concerne également les sels d'addition d'acide des dérivés de formule (I). Les acides mis en œuvre pour la préparation de ces sels pourront être des acides organiques tels que l'acide maléique, oxalique ou fumarique et des acides minéraux tels que l'acide chlorhydrique.

Entrent également dans le cadre de la présente invention, les hydrates des dérivés de formule (I) et des sels de ces derniers.

Entrent en outre dans le cadre de la présente invention, les N-oxydes des dérivés de formule (I).

Le procédé selon l'invention pour l'obtention des dérivés de formule (I) consiste, lorsque X représente un atome d'oxygène, à condenser :

soit les acides de formule :

$$Ar{-}COOH \qquad (II)$$

dans laquelle Ar a les mêmes significations que dans la formule (I),

soit les chlorures d'acide de formule :

$$Ar{-}COCl \qquad (III)$$

dans laquelle Ar a les mêmes significations que dans la formule (I), respectivement avec les dérivés amino quinuclidiniques de formule :

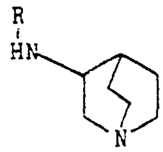

(IV)

dans laquelle R a les mêmes significations que dans la formule (I).

Dans les cas de l'utilisation des acides (II), la condensation est réalisée selon la technique dite de « BOISSONAS », de préférence en présence d'un chloroformiate d'alkyle et de triéthylamine ; dans le cas de l'utilisation des chlorures d'acide (III), la condensation est de préférence réalisée en présence d'un agent basique tel que la triéthylamine et en solution dans un solvant aprotique.

Les composés (IV) sont obtenus par hydrogénolyse catalytique de préférence par le palladium sur charbon à 10 % en milieu hydroalcoolique et en autoclave, des composés de formule :

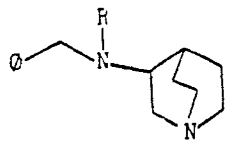

(V)

dans laquelle R a les mêmes significations que dans la formule (I).

Les composés (V) sont obtenus par une synthèse en deux stades qui consiste à condenser les aldéhydes de formule :

$R_5$—CHO (VI)

dans laquelle $R_5$ représente un groupe alkyle de 1 à 3 atomes de carbone ou l'aldéhyde formique, de préférence en milieu aqueux, avec la benzylamino-3 quinuclidine, puis à réduire le composé intermédiaire obtenu (non isolé) par le cyanoborohydrure de sodium ($NaBH_3CN$).

La benzylamino-3 quinuclidine est quant à elle obtenue par condensation du benzaldéhyde avec l'amino-3 quinuclidine (condensation effectuée de préférence dans le toluène) puis par réduction de la base de Schiff obtenue par le borohydrure de sodium ou de potassium (de préférence en milieu méthanolique).

Dans le cas où dans la formule (I) X représente l'atome d'oxygène et Ar représente un groupe phényle de structure particulière :

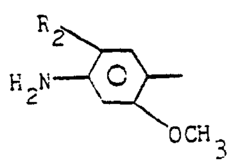

dans laquelle $R_2$ a les mêmes significations que précédemment, les dérivés (I) correspondants peuvent également être obtenus par hydrolyse acide (de préférence par l'acide chlorhydrique 2 N) des dérivés (I) de formule particulière :

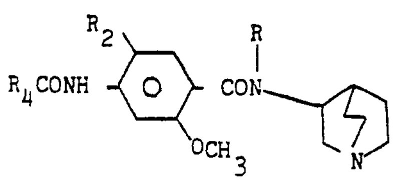

(Ia)

dans laquelle R et $R_2$ ont les mêmes significations que dans la formule (I) et $R_4$ représente un groupe alkyle de 1 à 4 atomes de carbone, les dérivés (Ia) étant obtenus conformément aux protocoles exposés précédemment.

Le procédé selon l'invention pour l'obtention des dérivés de formule (I) pour lequel X représente un atome de soufre, consiste à traiter les dérivés de formule (I) pour lesquels X représente l'atome d'oxygène :

soit par le réactif dit de « LAWESSON » et de formule :

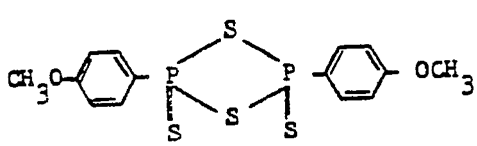

cette réaction étant effectuée en milieu aprotique et de préférence dans un mélange de benzène et de dioxanne,

soit par le pentasulfure de phosphore, de préférence dans l'H.M.P.T.

Les isomères optiques selon l'invention sont préparés à partir des mélanges racémiques correspondants. Plus précisément, ils sont obtenus en salifiant les dérivés racémiques de formule (I) sous forme de base, au moyen d'un acide organique optiquement actif, tel que l'acide L(d)-tartrique ou D(l)-tartrique, puis en dédoublant par la méthode des recristallisations fractionnées successives les sels résultant de ladite salification, lesdites recristallisations étant avantageusement effectuées dans le méthanol.

Les sels d'addition d'acide des dérivés de formule (I) peuvent être préparés par simple réaction des dérivés de formule (I) avec un acide organique ou minéral, selon les méthodes usuelles, les dérivés de formule (I) et/ou l'acide étant de préférence utilisés en solution dans des solvants appropriés.

Enfin, les N-oxydes des dérivés de formule (I) sont préparés selon les méthodes classiques décrites dans la littérature, soit à l'aide d'un peracide (M.C.P.B.A. de préférence) ou d'eau oxygénée.

Les préparations suivantes sont données à titre d'exemples pour illustrer l'invention.

Exemple 1

N-(quinuclidinyl-3) amino-4 chloro-5 méthoxy-2 benzamide, maléate (I)

Numéro de code : 2

A une solution refroidie à 0 °C de 12,1 g d'acide amino-4 chloro-5 méthoxy-2 benzoïque dans 300 ml de DMF, on ajoute 17,5 ml de triéthylamine, puis 4,6 ml de chloroformiate d'éthyle. On laisse agiter 45 minutes à température ambiante et on ajoute à cette solution une suspension de 12 g de dichlorhydrate de 3-aminoquinuclidine et de 13,2 ml de triéthylamine (qui a été agitée à 50 °C pendant une heure), à 0 °C et en 30 minutes. Puis on laisse 12 heures à température ambiante, évapore le DMF, reprend le résidu dans le chloroforme, lave avec une solution de carbonate de sodium, puis à l'eau, sèche sur sulfate de sodium, filtre, évapore le filtrat et chromatographie le résidu sur colonne d'alumine (Eluant : chloroforme-méthanol, 99 %-1 %). On dissout le composé obtenu (4,3 g) dans l'acétate d'éthyle et ajoute une solution de 1,57 g d'acide maléique dans 30 ml d'acétate d'éthyle et filtre le précipité obtenu (3,8 g).

Rendement : 15 %

Point de fusion : 194 °C

Par ce même procédé, mais à partir des réactifs correspondants, on obtient les composés de formule (I) de numéros de code 1, 3 à 8, 12, 15 à 18, 20, 21, 23 et 24 figurant dans le tableau I ci-après.

Exemple 2

N-(quinuclidinyl-3) fluoro-3 benzamide, oxalate (I)

Numéro de code : 5

On porte à 50 °C pendant une heure une suspension de 12 g de dichlorhydrate d'amino-3 quinuclidine et de 33,6 ml de triéthylamine dans 100 ml de DMF. Puis on refroidit à 0 °C et on ajoute une solution de 9,5 g de chlorure de l'acide fluoro-3 benzoïque dans 30 ml de diméthylformamide en une heure.

Puis on laisse agiter 12 heures à température ambiante, filtre, évapore le filtrat, reprend le résidu dans le chlorure de méthylène, lave avec une solution de carbonate de sodium, à l'eau, sèche sur sulfate de sodium, filtre et évapore le solvant. On dissout le résidu dans l'acétone et ajoute une solution de 5,4 g d'acide oxalique dans l'acétone, filtre le précipité obtenu et le recristallise dans l'éthanol à 88 %. On obtient 7,4 g (rendement = 38 %) du composé attendu.

Par le même procédé, mais à partir des réactifs correspondants, on obtient les composés de formule (I) figurant dans le tableau I ci-après et de numéros de code 1 à 4, 6 à 8, 12, 15 à 18, 20, 21, 23 et 24.

Exemple 3

chlorhydrate hydraté de l'(amino-4 chloro-5 méthoxy-2) phényl-1·N-(quinuclidinyl-3)-thioformamide (I)

Numéro de code : 22

On porte au reflux pendant 3 heures un mélange de 14,5 g d'(amino-4 chloro-5 méthoxy-2) benzoylamino-3 quinuclidine [(I) ; numéro de code 2] et de 27,1 g de réactif de LAWESSON dans 500 ml de benzène et 500 ml de dioxanne. Puis on lave à l'aide de NaOH concentrée, à l'eau, extrait les eaux de lavage à l'aide d'acétate d'éthyle, rassemble les phases organiques, les sèche sur sulfate de sodium, filtre et évapore le filtrat. On chromatographie le résidu sur une colonne d'alumine (éluant : acétate d'éthyle

90 %-méthanol 10 %). Le produit purifié obtenu est dissous dans l'éthanol, on ajoute de l'éthanol chlorhydrique à la solution obtenue, puis refroidit et filtre le précipité obtenu que l'on recristallise dans un mélange méthanol-eau (98 %-2 %). On obtient ainsi 1,8 g du produit attendu (Rendement = 12 %).

Exemple 4

Hémi-fumarate de l'(amino-4 chloro-5 méthoxy-2) N-(quinuclidinyl-3) N-méthyl benzamide (I)

Numéro de code : 24

On porte au reflux pendant 15 minutes une solution de 45,4 g d'(acétamido-4 chloro-5 méthoxy-2) N-(quinuclidinyl-3) N-méthyl benzamide [(I), numéro de code 23 — sous forme de base et préparée selon l'un quelconque des exemples 1 ou 2 précédents] dans 450 ml d'HCl 2N. Puis on lave à l'aide d'acétate d'éthyle, basifie à l'aide de soude concentrée, extrait au chloroforme, sèche sur sulfate de sodium (ou de magnésium), filtre et évapore le filtrat. Le résidu est dissous dans l'éthanol, on ajoute une solution éthanolique d'acide fumarique et filtre le précipité obtenu. On obtient 29,4 g (rendement = 62 %) du produit attendu.

Exemple 5

Hémi-fumarate de l'(amino-4 chloro-5 méthoxy-2) N-(quinuclidinyl-3) N-méthyl thiobenzamide (I)

Numéro de code : 25

On porte à 110 °C pendant 2 heures une solution de 12 g de pentasulfure de sodium et de 14,6 g du composé (I) obtenu à l'exemple précédent (sous forme de base) dans 150 ml d'H.M.P.T. Puis on jette la solution dans de l'eau glacée, filtre, reprend le précipité dans de l'eau basique (NaOH aqueuse) et extrait au chloroforme. On sèche sur sulfate de sodium (ou de magnésium), filtre et évapore le filtrat ; le résidu est chromatographié sur colonne d'alumine (éluant : chloroforme 99 %-méthanol 1 %), puis sur une colonne de silice (chromatographie liquide à moyenne pression ; éluant : chloroforme 95 %-méthanol 4,75 %-ammoniaque 0,25 %), ces chromatographies étant nécessaires pour éliminer l'H.M.P.T. On obtient ainsi 3,2 g (rendement = 21 %) de produit pur que l'on dissout dans l'éthanol, on ajoute une solution éthanolique d'acide fumarique et filtre le précipité obtenu qui correspond au produit attendu.

Exemple 6

méthylamino-3 quinuclidine (IV)

1er stade : benzylamino-3 quinuclidine

On porte au reflux pendant 45 minutes un mélange de 50 g d'amino-3 quinuclidine et de 40 ml de benzaldéhyde dans 1 000 ml de toluène, en éliminant l'eau formée. Puis on évapore le solvant, dissout le résidu dans 800 ml de méthanol et ajoute en 3 heures 15 g de borohydrure de potassium en maintenant la température à 0 °C. Puis on évapore le solvant, reprend le résidu dans l'eau, extrait à l'acétate d'éthyle (ou au chloroforme), sèche sur sulfate de sodium (ou de magnésium), filtre et évapore le filtrat. On obtient ainsi 85 g (rendement ≃ 100 %) du produit attendu pur (unique en C.C.M.) qui se présente sous forme liquide.

2ème stade : (N-méthyl N-benzylamino)-3 quinuclidine (V)

A une solution de 73,7 g de benzylamino-3 quinuclidine dans de l'eau (≃ 300 à 400 ml) on ajoute, en refroidissant à 5 °C, 51 ml d'une solution aqueuse de formaldéhyde à 40 %. Puis après l'addition, on laisse 30 minutes à 5 °C, puis on ajoute 32 g de cyanoborohydrure de sodium en 1 à 2 heures. Puis on laisse 12 heures à température ambiante, extrait à l'acétate d'éthyle, sèche sur sulfate de sodium (ou de magnésium), filtre, évapore le filtrat et distille le résidu ($Eb_{0,04mm/Hg}$ = 125 °C). On obtient ainsi pratiquement quantitativement le produit attendu.

3ème stade :

On porte à 80 °C, pendant 6 heures 30 minutes, sous une pression de 6 bars d'hydrogène en autoclave, un mélange de 33,3 g de (N-méthyl N-benzylamino)-3 quinuclidine, de 16 g de palladium sur charbon à 10 % (50 % d'humidité) dans 500 ml d'éthanol. Puis on filtre, évapore le solvant (sous bon vide et à froid) et distille le résidu ($Eb_{24mm/Hg}$ = 110 °C). On obtient ainsi le produit attendu avec un rendement de 43 %.

Exemple 7

chlorhydrate des méta-chloro (N-quinuclidinyl-3) benzamide dextrogyre (26) et lévogyre (27)

A 52,5 g de méta-chloro (N-quinuclidinyl-3) benzamide racémique [(I), numéro de code 20], sous forme de base, on ajoute une solution de 29,7 g d'acide tartrique L, dextrogyre, dans 500 ml de méthanol, on porte au reflux, filtre à chaud et laisse refroidir. On obtient, après filtration, 47 g de précipité (Point de fusion ≃ 178 °C), que l'on redissout dans 500 ml de méthanol bouillant. Après refroidissement et filtration, on obtient 28 g d'un précipité (Point de fusion ≃ 188 °C) que l'on dissout dans 250 ml de méthanol bouillant. Après refroidissement et filtration, on obtient 20 g de composé (Point de fusion ≃ 192 °C) qui est dissout dans l'eau, on basifie la solution aqueuse obtenue à l'aide de carbonate de sodium, extrait au chloroforme, sèche sur sulfate de sodium, filtre et évapore le filtrat. Le produit obtenu est dissout dans l'acétone et on ajoute de l'éthanol chlorhydrique (≃ 6N), on filtre le précipité obtenu et le recristallise dans l'éthanol. On obtient ainsi 9,4 g d'isomère dextrogyre : $\alpha_D^{20}$ (méthanol, C ≃ 2,5) = + 15,1°, point de fusion = 240 °C [la base correspondante a un $\alpha^0$ (méthanol, C ≃ 2,5) = + 33,3° et point de fusion = 125 °C]. Les eaux mères (filtrats) des trois premières recristallisations dans le méthanol sont rassemblées, évaporées, le résidu est repris dans l'eau, on basifie à l'aide de carbonate de sodium, extrait au chloroforme, sèche sur sulfate de sodium (ou de magnésium), filtre et évapore le filtrat. On obtient 42 g de produit auxquels on ajoute une solution de 23,8 g d'acide tartrique D, lévogyre dans 500 ml de méthanol ; on porte le mélange au reflux, filtre à chaud, puis après refroidissement du filtrat, filtre le précipité obtenu (Point de fusion = 186 °C). Ce précipité est dissout dans 350 ml de méthanol bouillant, on filtre à chaud, puis après refroidissement du filtrat, on filtre le précipité obtenu. On obtient ainsi 25 g d'un précipité (Point de fusion = 192 °C) que l'on dissout dans l'eau. On basifie à l'aide de carbonate de sodium, extrait au chloroforme, sèche sur sulfate de sodium (ou de magnésium), filtre et évapore le filtrat, le résidu est dissout dans l'acétone et on ajoute de l'éthanol chlorhydrique (≃ 6 N). On filtre le précipité obtenu et le recristallise dans l'éthanol (≃ 100 ml). On obtient ainsi 10,9 g d'isomère lévogyre : $\alpha_D^{20}$ (méthanol ; C ≃ 2,5) = —15,4°, point de fusion = 240 °C [la base correspondante a un $\alpha_D^{20}$ (méthanol, C ≃ 2,5) = — 34,3° et point de fusion = 125 °C).

(Voir Tableau I pages suivantes)

Tableau I

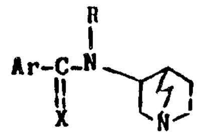

(I)

| Numéro de Code | Ar - C -<br>X | R | Forme | Formule brute | Poids moléculaire | Point de fusion (°C) | ANALYSE ELEMENTAIRE % | C | H | N |
|---|---|---|---|---|---|---|---|---|---|---|
| 1 | 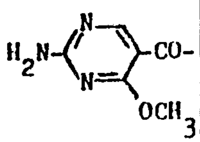 | H | Base hydratée | $C_{13}H_{19}N_5O_2$ + 0,5 % $H_2O$ | 277,32 | 198 | Cal. | 55,97 | 6,82 | 25,12 |
| | | | | | | | Tr. | 55,61 | 6,70 | 25,43 |
| 2 | 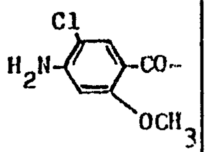 | " | Maléate | $C_{19}H_{24}ClN_3O_6$ | 425,86 | 194 | Cal. | 53,58 | 5,68 | 9,87 |
| | | | | | | | Tr. | 53,32 | 5,78 | 9,97 |
| 3 | 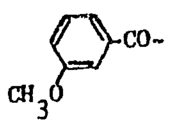 | " | Oxalate | $C_{17}H_{22}N_2O_6$ | 350,36 | 188 | Cal. | 58,27 | 6,33 | 8,00 |
| | | | | | | | Tr. | 58,15 | 6,47 | 7,89 |
| 4 | 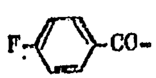 | " | " | $C_{16}H_{19}FN_2O_5$ | 338,33 | 232 | Cal. | 56,80 | 5,66 | 8,28 |
| | | | | | | | Tr. | 56,52 | 5,66 | 8,27 |

Tableau I (Suite)

| Numéro de Code | Ar – C(=X) – | R | Forme | Formule brute | Poids moléculaire | Point de fusion (°C) | ANALYSE ELEMENTAIRE % | C | H | N |
|---|---|---|---|---|---|---|---|---|---|---|
| 5 | 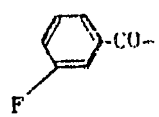 | H | Oxalate | $C_{16}H_{19}FN_2O_5$ | 338,33 | 223 | Cal. | 56,80 | 5,66 | 8,28 |
| | | | | | | | Tr. | 56,73 | 5,72 | 8,27 |
| 6 | 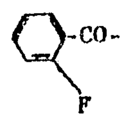 | " | HCl | $C_{14}H_{18}FClN_2O$ | 284,76 | 238 | Cal. | 59,05 | 6,37 | 9,84 |
| | | | | | | | Tr. | 58,97 | 6,35 | 9,84 |
| 7 | 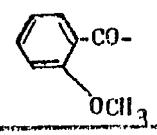 | " | Oxalate | $C_{17}H_{22}N_2O_6$ | 350,36 | 188 | Cal. | 58,27 | 6,33 | 8,00 |
| | | | | | | | Tr. | 58,29 | 6,55 | 8,12 |
| 8 | 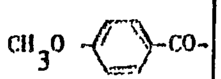 | " | HCl hydraté | $C_{15}H_{21}ClN_2O_2$ + $H_2O$ | 314,84 | 190 | Cal. | 57,23 | 7,36 | 8,90 |
| | | | | | | | Tr. | 57,47 | 6,92 | 8,83 |

Tableau I (Suite)

| Numéro de Code | 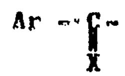 | R | Forme | Formule brute | Poids moléculaire | Point de fusion (°C) | ANALYSE ELEMENTAIRE % | C | H | N |
|---|---|---|---|---|---|---|---|---|---|---|
| | | | | | | | Cal. | | | |
| | | | | | | | Tr. | | | |
| 12 | 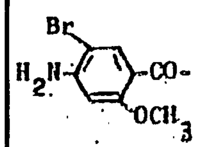 | H | HCl | $C_{15}H_{21}BrClN_3O_2$ | 389,70 | > 260 | Cal. | 46,11 | 5,42 | 10,76 |
| | | | | | | | Tr. | 45,69 | 5,35 | 10,51 |
| 15 | 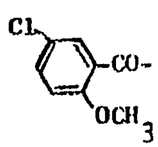 | " | " | $C_{15}H_{20}ClN_2O_2$ | 331,24 | 215 | Cal. | 54,39 | 6,09 | 8,46 |
| | | | | | | | Tr. | 54,07 | 6,25 | 8,33 |
| 16 | 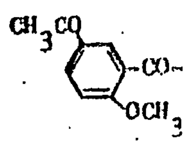 | " | " | $C_{17}H_{23}ClN_2O_3$ | 338,83 | 245 | Cal. | 60,26 | 6,84 | 8,27 |
| | | | | | | | Tr. | 60,03 | 6,91 | 8,20 |

Tableau I (Suite)

| Numéro de Code | Ar - C- ‖ X | R | Forme | Formule brute | Poids molé-culaire | Point de fusion (°C) | ANALYSE ELEMENTAIRE % | C | H | N |
|---|---|---|---|---|---|---|---|---|---|---|
| 17 | 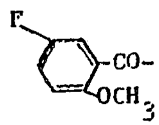 | H | HCl | $C_{15}H_{20}ClFN_2O_2$ | 314,78 | 246 | Cal. | 57,23 | 6,40 | 8,90 |
| | | | | | | | Tr. | 57,36 | 6,31 | 8,92 |
| 18 | 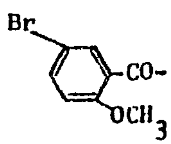 | " | " | $C_{15}H_{20}ClBrN_2O_2$ | 375,69 | 220 | Cal. | 47,95 | 5,37 | 7,46 |
| | | | | | | | Tr. | 47,80 | 5,58 | 7,48 |
| | | | | | | | Cal. | | | |
| | | | | | | | Tr. | | | |
| 20 | 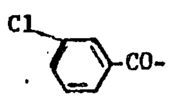 | " | HCl | $C_{14}H_{18}Cl_2N_2O$ | 301,21 | 242 | Cal. | 55,82 | 6,02 | 9,30 |
| | | | | | | | Tr. | 55,53 | 6,08 | 9,35 |

Tableau I (Suite)

| Numéro de Code | $Ar - \underset{\parallel X}{C} -$ | R | Forme | Formule brute | Poids moléculaire | Point de fusion (°C) | ANALYSE ELEMENTAIRE % | C | H | N |
|---|---|---|---|---|---|---|---|---|---|---|
| 21 | 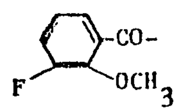 | H | Base | $C_{15}H_{19}FN_2O_2$ | 278,32 | 125 | Cal. | 64,73 | 6,88 | 10,07 |
| | | | | | | | Tr. | 64,47 | 7,00 | 10,00 |
| 22 | 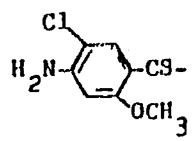 | H | 1,05 HCl +0,5 % $H_2O$ | $C_{15}H_{21}Cl_2N_3OS$ + 0,5 % $H_2O$ + 0,05 HCl | 365,97 | > 260 | Cal. | 49,22 | 5,86 | 11,48 |
| | | | | | | | Tr. | 48,26 | 5,54 | 11,51 |
| 23 | 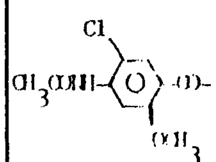 | $CH_3$ | Maléate | $C_{22}H_{28}ClN_3O_7$ | 481,92 | 237 | Cal. | 54,82 | 5,85 | 8,72 |
| | | | | | | | Tr. | 54,96 | 6,07 | 8,43 |
| 24 | 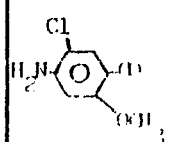 | " | ½ fumarate +1,35% $H_2O$ | $C_{18}H_{24}ClN_3O_4$ + 1,35 % $H_2O$ | 387,08 | > 260 | Cal. | 55,85 | 6,40 | 10,86 |
| | | | | | | | Tr. | 55,79 | 6,29 | 10,50 |

Tableau I (Suite)

| Numéro de Code | 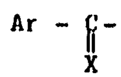 | R | Forme | Formule brute | Poids moléculaire | Point de fusion (°C) | ANALYSE ELEMENTAIRE % | C | H | N |
|---|---|---|---|---|---|---|---|---|---|---|
| 25 | 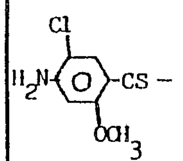 | $CH_3$ | ½ fumarate +11,7% $H_2O$ | $C_{18}H_{24}ClN_3O_3S$ + 11,7 % $H_2O$ | 444,32 | 194 | Cal. | 48,00 | 6,67 | 9,33 |
| | | | | | | | Tr. | 47,83 | 6,00 | 9,35 |
| 26 | 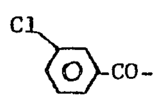 | H | HCl dextrogyre | $C_{14}H_{18}Cl_2N_2O$ | 301,21 | 240 | Cal. | 55,82 | 6,02 | 9,30 |
| | | | | | | | Tr. | 55,70 | 6,18 | 9,39 |
| | | | Base dextrogyre | $C_{14}H_{17}ClN_2O$ | 264,75 | 125 | Cal. | - | - | - |
| | | | | | | | Tr. | - | - | - |
| 27 | 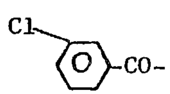 | H | HCl lévogyre | $C_{14}H_{18}Cl_2N_2O$ | 301,21 | 240 | Cal. | 55,82 | 6,02 | 9,30 |
| | | | | | | | Tr. | 55,71 | 6,20 | 9,27 |
| | | | Base lévogyre | $C_{14}H_{17}ClN_2O$ | 264,75 | 125 | - | - | - | - |
| | | | | | | | Cal. | | | |
| | | | | | | | Tr. | | | |

Les composés selon l'invention ont été étudiés chez l'animal de laboratoire et ont montré une activité sur le système digestif (notamment comme accélérateurs de la motricité gastro-intestinale). Cette activité a été mise en évidence :

par le test de l'évacuation gastrique chez le rat, réalisé selon le protocole suivant : on administre par voie orale, chez des rats vigiles à jeun depuis 20 heures les composés selon l'invention simultanément à 20 billes d'acier. L'action sur l'évacuation gastrique des composés testés est appréciée 90 minutes après leur administration par le pourcentage d'animaux dont l'estomac ne contient plus aucune bille, ces billes étant dénombrées par examen radiologique. Pour illustrer l'invention, on a rassemblé dans le tableau II ci-après quelques résultats obtenus avec les composés selon l'invention ;

par le test du transit total chez le rat, réalisé selon le protocole suivant : on administre par voie orale, chez des rats vigiles à jeun depuis 20 heures les composés selon l'invention simultanément à 20 billes d'acier. Le jeûne est levé immédiatement après l'administration et les composés selon l'invention sont administrés une seconde fois à la même dose après un délai de 7 heures. Le transit total est apprécié 24 heures après la première administration des composés à tester, par le pourcentage d'animaux ayant évacué la totalité des billes dans les fèces, les billes étant dénombrées par examen radiologique des rats et des fèces. Pour illustrer l'invention, on a rassemblé dans le tableau III ci-après quelques résultats obtenus avec les composés selon l'invention.

Par ailleurs, la toxicité aiguë est évaluée chez la souris selon la méthode de MILLER et TAINTER décrite dans Proc. Soc. Exp. Biol. Med. 57, 261, (1944).

Tableau II

| Numéro de code des composés testés | Toxicité $DL_{50}$ - souris (mg/kg/i.p.) | Dose administrée (mg/kg/p.o.) | % d'animaux dont l'estomac ne contient plus de bille après 90 minutes |
|---|---|---|---|
| 2 | 105 | 5<br>10 | 75<br>100 |
| 3 | 110 | 2,5 | 100 |
| 5 | 140 | 2,5 | 100 |

Tableau III

| Numéro de code des composés testés | Toxicité $DL_{50}$ - souris (mg/kg/i.p.) | Dose administrée (mg/kg/p.o.) | % d'animaux ayant totalement évacué les billes après 24 heures |
|---|---|---|---|
| 2 | 105 | 1 | 64 |
| 7 | 150 | 10 | 50 |
| 6 | 150 | 10 | 55 |
| 17 | 140 | 1 | 57 |
| 18 | 160 | 1 | 38 |
| 22 | 300 | 1 | 63 |

Tableau III (Suite)

| Numéro de code des composés testés | Toxicité $DL_{50}$ - souris (mg/kg/i.p.) | Dose administrée (mg/kg/p.o.) | % d'animaux ayant totalement évacué les billes après 24 heures |
|---|---|---|---|
| 1 | 300 | 10 | 50 |
| 20 | 145 | 1 | 50 |
| 23 | 50 | 1 | 38 |
| 24 | 70 | 1 | 38 |
| 25 | 80 | 1 | 44 |

Comme le montrent les tableaux ci-dessus, l'écart entre les doses toxiques et les doses actives permet l'emploi en thérapeutique des composés selon l'invention dans le domaine du système digestif et plus particulièrement comme accélérateur de la motricité gastro-intestinale et comme potentialisateur de médicament, notamment de médicaments analgésiques comme l'aspirine ou le paracétamol par exemple.

La présente invention a donc également pour objet, à titre de médicaments, et notamment à titre de médicaments utiles dans le domaine du système digestif, les dérivés de formule (I), leurs sels d'addition d'acide pharmaceutiquement acceptables, les hydrates de ces dérivés et sels et les N-oxydes de ces dérivés.

L'invention s'étend enfin aux compositions pharmaceutiques renfermant, comme principe actif, au moins un des médicaments définis ci-dessus, en association avec un véhicule approprié. Ces compositions pourront être administrées par voie orale, sous forme de gélules, comprimés, dragées etc... à des doses pouvant atteindre 500 mg de principe actif par jour (en une ou plusieurs prises par jour) ou par voie parentérale sous forme de solutés injectables à des doses pouvant atteindre 200 mg de principe actif par jour (en une ou deux injections journalières).

**Revendications**

1. Dérivés de l'amino-3 quinuclidine répondant à la formule générale :

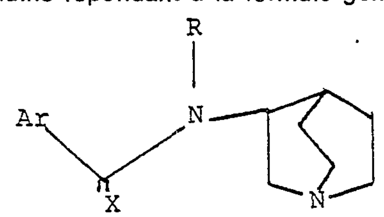

(I)

dans laquelle X représente un atome de soufre ou d'oxygène, R désigne un atome d'hydrogène, un groupe alkyle de 1 à 4 atomes de carbone ou le groupe benzyle, et Ar désigne :

un noyau phényle substitué par un atome d'halogène ou un groupe méthoxy,

un noyau phényle de structure

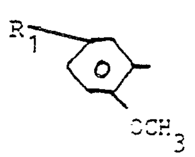

dans laquelle $R_1$ représente un atome d'halogène ou un groupe alkylcarbonyle dont le reste alkyle comporte de 1 à 4 atomes de carbone,

le groupe (fluoro-3 méthoxy-2) phényle,

le groupe (amino-2 méthoxy-4) pyrimidinyle-5, ou

un noyau phényle de structure

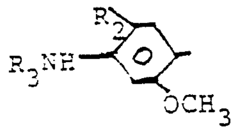 ,

où $R_2$ représente un atome d'halogène et $R_3$ représente un atome d'hydrogène ou un groupe alkylcarbonyle dont le reste alkyle comporte de 1 à 4 atomes de carbone,
ainsi que les isomères optiques dextrogyre ou lévogyre correspondant à ces dérivés, les sels d'addition d'acide organique ou minéral de ces dérivés et isomères optiques, les hydrates de ces dérivés, isomères optiques et sels et les N-oxydes correspondant à ces dérivés et isomères optiques.

2. Composés selon la revendication 1, pour lesquels le couple (X, R) prend la valeur (O, H) et Ar prend l'une quelconque des valeurs suivantes :

( 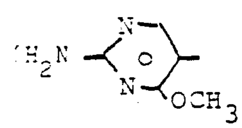 ) ; ( 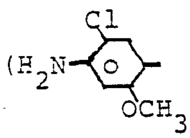 ) ; ( 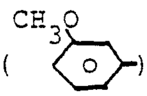 ) ; ( 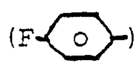 ) ; ( 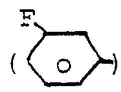 ) ;

( 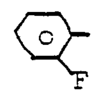 ) ; ( 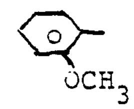 ) ; ( 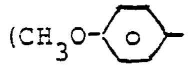 ) ; ( 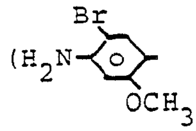 ) ; ( 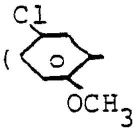 ) ;

( 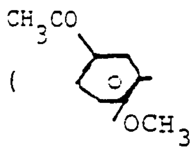 ) ; ( 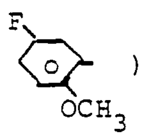 ) ; ( 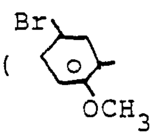 ) ; ( 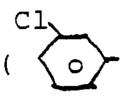 ) ;

( 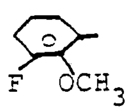 ) ; ( 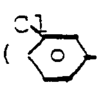 , dextrogyre ( 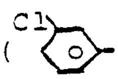 , lévogyre

3. Composés selon la revendication 1, pour lesquels le couple (X, R) prend la valeur (O, $CH_3$) et Ar prend l'une des valeurs suivantes :

( 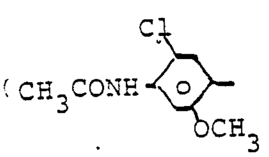 ) ; ( 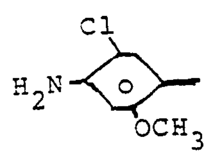 ).

4. Composés selon la revendication 1, pour lesquels le couple (X, R) prend la valeur (S, H) ou (S, $CH_3$) et Ar prend la valeur

( 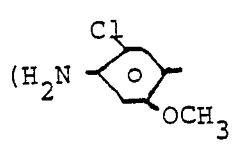 )

5. Médicament caractérisé en ce qu'il est constitué par l'un quelconque des composés objet des revendications 1 à 4.

6. Composition pharmaceutique, caractérisée en ce qu'elle est constituée par au moins l'un des médicaments selon la revendication 5, en association avec un véhicule pharmaceutiquement acceptable.

7. Procédé de préparation des composés de formule (I) selon la revendication 1 sous forme de base pour lesquels X désigne un atome d'oxygène, caractérisé en ce qu'il consiste à condenser les acides de formule :

$$Ar—COOH \qquad (II)$$

dans laquelle Ar a les mêmes significations que dans la formule (I) par la méthode dite de « BOISSONNAS » respectivement avec les dérivés de l'amino-3 quinuclidine de formule :

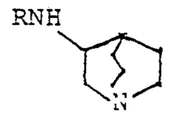

(IV)

dans laquelle R représente l'atome d'hydrogène ou un groupe alkyle de 1 à 4 atomes de carbone ou benzyle.

8. Procédé de préparation des composés de formule (I) selon la revendication 1 sous forme de base pour lesquels X désigne un atome d'oxygène, caractérisé en ce qu'il consiste à condenser un chlorure d'acide de formule :

Ar—COCl (III)

dans laquelle Ar a les mêmes significations que dans la formule (I) avec les composés de formule (IV) définie à la revendication 7.

9. Procédé selon la revendication 8, caractérisé en ce que la réaction est réalisée en milieu aprotique et en présence d'un agent basique tel que la triéthylamine.

10. Procédé de préparation des composés de formule (I) selon la revendication 1 sous forme de base pour lesquels X représente un atome de soufre, caractérisé en ce qu'il consiste à traiter les composés de formule (I) pour lesquels X représente l'atome d'oxygène, soit par le réactif de « LAWESSON » de formule :

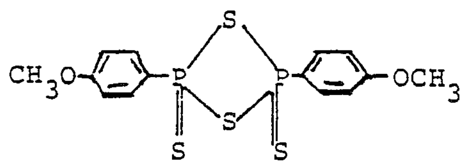

en milieu aprotique et de préférence dans un mélange de benzène et de dioxanne, soit par le pentasulfure de phosphore, de préférence dans l'H.M.P.T.

11. Procédé de préparation des composés de formule (I) selon la revendication 1 sous forme de base pour lesquels X représente un atome d'oxygène et Ar prend la valeur particulière

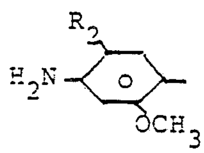

dans laquelle $R_2$ a les mêmes significations que dans la formule (I), caractérisé en ce qu'il consiste en une hydrolyse acide des composés (I) de formule particulière :

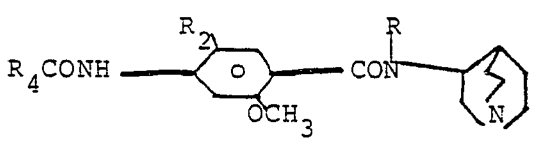

(Ia)

dans laquelle R et $R_2$ ont les mêmes significations que dans la formule (I) et $R_4$ représente un groupe alkyle de 1 à 4 atomes de carbone.

12. Procédé de préparation des isomères optiques dextrogyre et lévogyre sous forme base des composés de formule (I) selon la revendication 1, caractérisé en ce qu'il consiste à salifier les composés racémiques de formule (I) sous forme de base, au moyen d'un acide organique optiquement actif, puis à dédoubler par la méthode des recristallisations fractionnées successives les sels résultant de cette salification.

13. Procédé selon la revendication 12, caractérisé en ce que l'acide optiquement actif est l'acide L (dextrogyre) tartrique ou l'acide D (lévogyre) tartrique.

14. Procédé selon la revendication 12 ou 13, caractérisé en ce que les recristallisations fractionnées sont effectuées dans le méthanol.

**Claims**

1. 3-amino quinuclidine derivatives corresponding to the general formula :

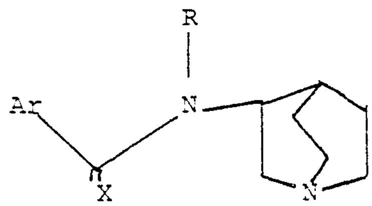

(I)

in which X represents a sulfur or oxygen atom, R designates a hydrogen atom, an alkyl group with 1 to 4 carbon atoms or the benzyl group, and Ar designates :

a phenyl nucleus substituted by a halogen atom or a methoxy group,

a phenyl nucleus of structure

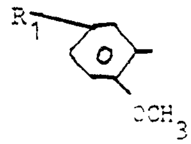

in which $R_1$ represents a halogen atom or an alkylcarbonyl group whose alkyl residue comprises 1 to 4 carbon atoms,

the (3-fluoro 2-methoxy) phenyl group,

the (2-amino 4-methoxy) 5-pyrimidinyl group, or

a phenyl nucleus of structure

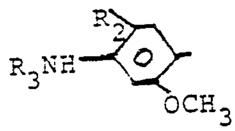 ,

in which $R_2$ represents a halogen atom and $R_3$ represents a hydrogen atom or an alkylcarbonyl group whose alkyl residue comprises 1 to 4 carbon atoms,

as well as the dextrorotatory or levorotatory optical isomers coresponding to these derivatives, the organic or mineral acid addition salts of these derivatives and optical isomers, the hydrates of these derivatives, optical isomers and salts and the N-oxides corresponding to these derivatives and optical isomers.

2. The compounds as claimed in claim 1, for which the pair (X, R) takes the value (O, H) and Ar takes any one of the following values :

( 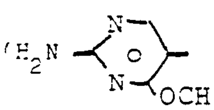 ) ; ( 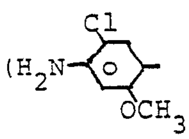 ) ; ( 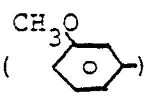 ) ; ( 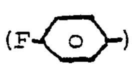 ) ; ( 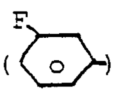 ) ;

( 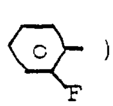 ) ; ( 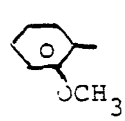 ) ; ( 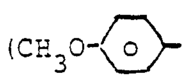 ) ; ( 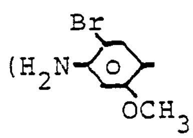 ) ; ( 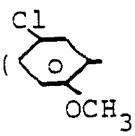 ) ;

( 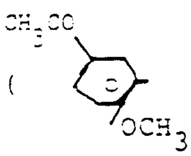 ) ; ( 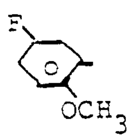 ) ; ( 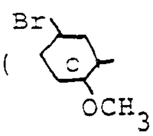 ) ; ( 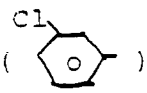 ) ;

( 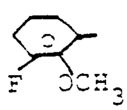 ) ; ( 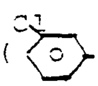 , dextrorotatory ( 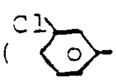 , levorotatory

3. The compounds as claimed in claim 1, for which the pair (X, R) takes the value (O, $CH_3$) and Ar takes any one of the following values :

( 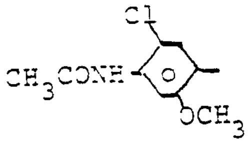 ) ; ( 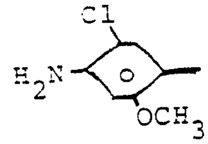 ).

4. The compounds as claimed in claim 1, for which the pair (X, R) takes the value (S, H) or (S, $CH_3$) and Ar takes the value

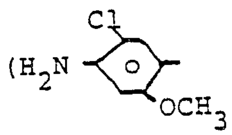

5. A drug, characterized in that it is formed by any one of the compounds claimed in claims 1 to 4.

6. A pharmaceutical composition, characterized in that it is formed by at least one of the drugs as claimed in claim 5, in association with a pharmaceutically acceptable vehicle.

7. A process for preparing the compounds of formula (I) according to claim 1 in base form for which X designates an oxygen atom, characterized in that it consists in condensing the acids or formula :

Ar—COOH (II)

in which Ar has the same meanings as in formula (I) by the so-called « BOISSONNAS » method respectively with the derivatives of 3-amino quinuclidine of formula :

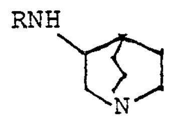

(IV)

in which R represents the hydrogen atom, an alkyl group with 1 to 4 carbon atoms or a benzyl group.

8. A process for preparing the compounds of formula (I) according to claim 1 in base form for which X designates an oxygen atom, characterized in that it consists in condensing an acid chloride of formula :

Ar—COCl (III)

in which Ar has the same meanings as in formula (I) with the compounds of formula (IV) defined in claim 7.

9. The process as claimed in claim 8, characterized in that the reaction is carried out in an aprotic medium and in the presence of a basic agent such as triethylamine.

10. A process for preparing the compounds of formula (I) according to claim 1 in base form for which X represents a sulfur atom, characterized in that it consists in treating the compounds of formula (I) for which X represents the oxygen atom, either by means of the « LAWESSON »-reagent of formula :

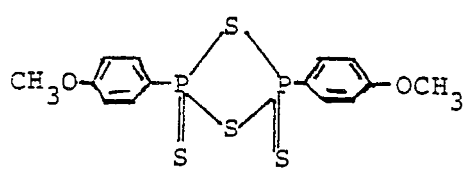

in an aprotic medium and preferably in a mixture of benzene and dioxane, or by means of phosphorous pentasulfine, preferably in H.M.P.T.

11. A process for preparing the compounds of formula (I) according to claim 1 in base form for which X represents an oxygen atom and Ar takes the particular value

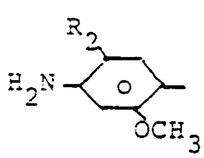

in which $R_2$ has the same meanings as in formula (I), characterized in that it consists in an acid hydrolysis of the compounds (I) of the particular formula :

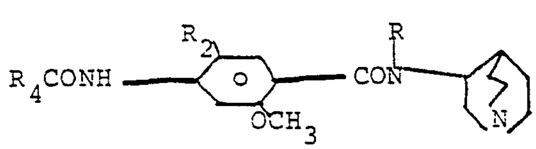

(Ia)

in which R and $R_2$ have the same meanings as in formula (I) and $R_4$ represents an alkyl group with from 1 to 4 carbon atoms.

12. A process for preparing the dextrorotatory and levorotatory optical isomers in base form of the compounds of formula (I) according to claim 1, characterized in that it consists in salifying the racemic compounds of formula (I) in base form by means of an optically active organic acid, then in resolving the salts resulting from this salification by the method of fractionated recrystallizations.

13. The process as claimed in claim 12, characterized in that the optically active acid is dextrorotatory L tartaric acid or levorotatory D tartaric acid.

14. The process as claimed in claim 12 or 13, characterized in that the fractionated recrystallizations are carried out in methanol.

**Patentansprüche**

1. Derivate des Amino-3 Quinuclidin mit der Generalformel :

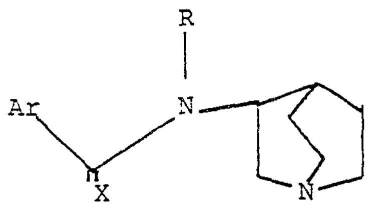 (I)

wobei X ein Schwefel- oder Sauerstoffatom ist, R ein Wasserstoffatom, eine Alkylgruppe mit 1 bis 4 Kohlenstoffatomen oder die Benzylgruppe bezeichnet und Ar definiert ist als

ein Phenylkern substituiert durch ein Halogenatom oder eine Methoxygruppe,

ein Phenylkern der Formel

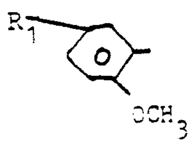

in welcher $R_1$ ein Halogenatom oder eine Alkylcarbonylgruppe bildet, deren Restalkyl 1 bis 4 Kohlenstoffatome umfaßt,

die (Fluor-3 Methoxy-2) Phenylgruppe,

die (Amino-2 Methoxy-4) Pyrimidinyl-5-Gruppe, oder

ein Phenylkern der Formel

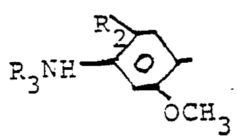 .

wobei $R_2$ ein Halogenatom und $R_3$ ein Wasserstoffatom oder eine Alkylcarbonylgruppe bildet, deren Restalkyl 1 bis 4 Kohlenstoffatome umfaßt,
sowie die optisch rechts- oder linksdrehenden Isomere, welche diesen Derivaten entsprechen, die Zusatzsalze der organischen oder mineralischen Säure dieser Derivate und optischen Isomere, die Hydrate dieser Derivate, optischen Isomere und Salze und die N-Oxide, welchen diesen Derivaten und optischen Isomeren entsprechen.

2. Verbindungen nach Anspruch 1, bei denen das Paar (X, R) den Wert (O, H) und Ar einen der folgenden Werte annimmt :

(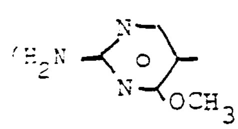) ; (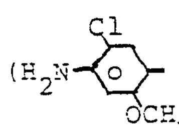) ; (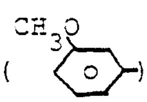) ; (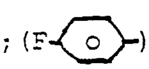) ; (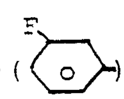) ;

(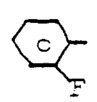) ; (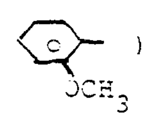) ; (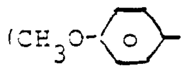) ; (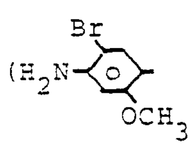) ; (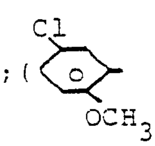) ;

( 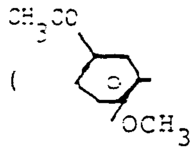 ); ( 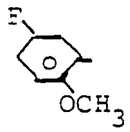 ); ( 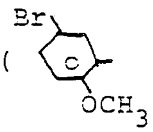 ); ( 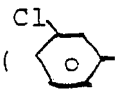 );

( 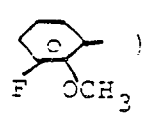 ); ( 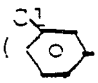 , rechtsdrehend ( 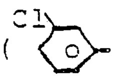 , linksdrehend

3. Verbindungen nach Anspruch 1, bei denen das Paar (X, R) den Wert (O, $CH_3$) und Ar einen der folgenden Werte annimmt :

( 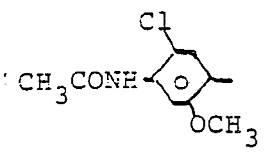 ); ( 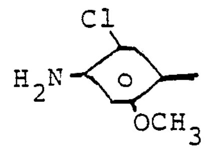 ).

4. Verbindungen nach Anspruch 1, bei denen das Paar (X, R) den Wert (S, H) oder (S, $CH_3$) und Ar den Wert

( 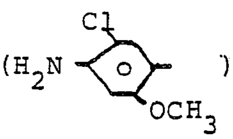 )

annimmt.

5. Arzneimittel, dadurch gekennzeichnet, daß es aus einer der Verbindungen gemäß den Ansprüchen 1 bis 4 besteht.

6. Pharmazeutische Mischung, dadurch gekennzeichnet, daß sie aus wenigstens einem der Arzneimittel gemäß Anspruch 5 besteht, in Verbindung mit einem pharmazeutisch akzeptierbaren Träger.

7. Verfahren zum Herstellen der Verbindung der Formel (I) gemäß Anspruch 1 als Base, wenn X ein Sauerstoffatom ist, dadurch gekennzeichnet, daß die Säuren der Formel :

Ar—COOH (II)

nach dem « BOISSONNAS » Verfahren kondensiert werden, wobei Ar dieselben Bedeutungen wie in der Formel (I) hat, zusammen mit den Derivaten des Amino-3 Quinuclidin der Formel :

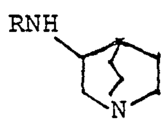 (IV)

wobei R das Wasserstoffatom oder eine Alkylgruppe mit 1 bis 4 Kohlenstoffatomen oder Benzyl ist.

8. Verfahren zum Herstellen der Verbindung der Formel (I) gemäß Anspruch 1 als Base, wenn X ein Sauerstoffatom ist, dadurch gekennzeichnet, daß eine Chlorsäure der Formel :

Ar—COCl (III)

kondensiert wird, wobei Ar dieselben Bedeutungen wie in der Formel (I) hat, zusammen mit den Verbindungen der Formel (IV) gemäß Anspruch 7.

9. Verfahren nach Anspruch 8, dadurch gekennzeichnet, daß die Reaktion in einem aprotischen Mittel und in Anwesenheit eines basischen Mittels wie Triäthylamin durchgeführt wird.

10. Verfahren zum Herstellen der Verbindung der Formel (I) gemäß Anspruch 1 als Base, wenn X ein Schwefelatom ist, dadurch gekennzeichnet, daß die Verbindungen der Formel (I), in denen X das Sauerstoffatom ist, entweder mit dem « LAWESSON »-Reagens der Formel :

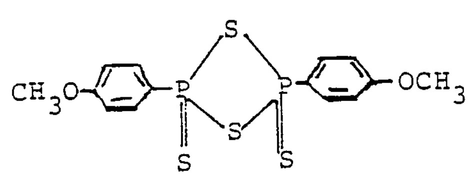

in einem aprotischen Mittel und vorzugsweise in einer Mischung aus Benzen und Dioxan behandelt werden oder mit Phosphorpentasulfid, vorzugsweise in H.M.P.T.

11. Verfahren zum Herstellen der Verbindungen der Formel (I) gemäß Anspruch 1 als Base, wenn X ein Sauerstoffatom ist und Ar den Wert

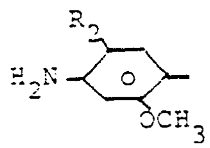

annimmt, wobei $R_2$ dieselben Bedeutungen wie in der Formel (I) hat, dadurch gekennzeichnet, daß es aus einer Säurehydrolyse der Verbindungen (I) mit der speziellen Formel :

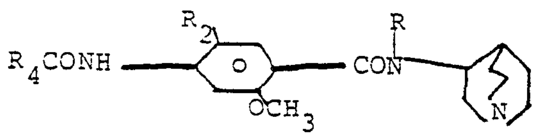

(Ia)

besteht, wobei R und $R_2$ dieselben Bedeutungen wie in der Formel (I) haben und $R_4$ eine Alkylgruppe mit 1 bis 4 Kohlenstoffatomen bildet.

12. Verfahren zum Herstellen der optisch rechts- und linksdrehenden Isomere als Base der Verbindungen der Formel (I) gemäß Anspruch 1, dadurch gekennzeichnet, daß die Salze der razemischen Verbindungen der Formel (I) als Base unter Verwendung einer optisch aktiven, organischen Säure hergestellt und dann die daraus resultierenden Salze mittels des Verfahrens der aufeinanderfolgenden, fraktionierten Rekristallisierungen entdoppelt werden.

13. Verfahren nach Anspruch 12, dadurch gekennzeichnet, daß die optisch aktive Säure die (rechtsdrehende) L-Traubensäure oder die (linksdrehende) D-Traubensäure ist.

14. Verfahren nach Anspruch 12 oder 13, dadurch gekennzeichnet, daß die fraktionierten Rekristallisierungen in Methanol durchgeführt werden.